(19) Europäisches Patentamt
European Patent Office
Office européen des brevets

(11) **EP 4 099 889 B1**

(12) **FASCICULE DE BREVET EUROPEEN**

(45) Date de publication et mention de la délivrance du brevet:
**03.04.2024 Bulletin 2024/14**

(21) Numéro de dépôt: **21707164.6**

(22) Date de dépôt: **02.02.2021**

(51) Classification Internationale des Brevets (IPC):
**A61B 3/15** *(2006.01)*  **A61B 1/00** *(2006.01)*
**A61B 3/10** *(2006.01)*  **A61B 3/113** *(2006.01)*
**G06F 3/01** *(2006.01)*

(52) Classification Coopérative des Brevets (CPC):
**A61B 3/152; A61B 3/102; A61B 3/113**

(86) Numéro de dépôt international:
**PCT/EP2021/052441**

(87) Numéro de publication internationale:
**WO 2021/156259 (12.08.2021 Gazette 2021/32)**

(54) **PROCÉDÉS ET SYSTÈMES DE CONTRÔLE DE L'ALIGNEMENT DE L'OEIL DANS UN APPAREIL D'IMAGERIE OPHTALMOLOGIQUE**

VERFAHREN UND SYSTEME ZUR INSPEKTION DER AUSRICHTUNG DES AUGES IN EINEM OPHTHALMOLOGISCHEN BILDERGEBUNGSGERÄT

METHODS AND SYSTEMS FOR INSPECTING THE ALIGNMENT OF THE EYE IN AN OPHTHALMOLOGICAL IMAGING APPARATUS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.02.2020 FR 2001067**

(43) Date de publication de la demande:
**14.12.2022 Bulletin 2022/50**

(73) Titulaire: **Imagine Eyes**
**91400 Orsay (FR)**

(72) Inventeurs:
• **LEFAUDEUX, Nicolas**
**91470 Forges-les-Bains (FR)**
• **LEVECQ, Xavier**
**91190 Gif sur Yvette (FR)**

(74) Mandataire: **Osha BWB**
**2, rue de la Paix**
**75002 Paris (FR)**

(56) Documents cités:
EP-A1- 2 281 500     EP-A1- 3 305 176
EP-B1- 2 281 500     US-A- 5 889 576
US-A1- 2007 159 599   US-B2- 9 213 163
US-B2- 10 369 053

• APPLEGATE R A ET AL: "Importance of fixation, pupil center, and reference axis in ocular wavefront sensing, videokeratography, and retinal image quality", JOURNAL CATARACT AND REFRACTIVE SURGERY, SURGERY, FAIRFAX, VA, US, vol. 35, no. 1, 1 janvier 2009 (2009-01-01), pages 139-152, XP025815165, ISSN: 0886-3350, DOI: 10.1016/J.JCRS.2008.09.014 [extrait le 2008-12-18]

EP 4 099 889 B1

## Description

## Domaine technique de l'invention

La présente description concerne des procédés et systèmes de contrôle de l'alignement de l'oeil dans un appareil d'imagerie ophtalmologique et concerne plus précisément le contrôle de la position de la pupille de l'oeil dans un référentiel d'un système d'imagerie dudit appareil.

## Etat de la technique

Des exemples d'appareils d'imagerie ophtalmologiques comprennent des appareils de tomographie par cohérence optique (ou OCT, abréviation de l'expression anglo-saxonne « Optical Cohérence Tomography ») pour capturer des images tomographiques de segments antérieurs (e.g. cornée) ou postérieurs (e.g. rétine) de l'oeil, des caméras pour l'imagerie du fond de l'oeil, des ophtalmoscopes à balayage laser (ou SLO pour « Scanning Laser Ophtalmoscope ») pour capturer des images du fond de l'oeil par balayage d'un faisceau laser à l'aide d'un système optique confocal, des microscopes à lampe à fente, etc. Dans tous ces appareils d'imagerie, un contrôle de l'alignement de l'oeil s'avère nécessaire, car des mouvements involontaires de l'oeil entraînent des décalages de la pupille de l'oeil par rapport au système d'imagerie et de ce fait des imprécisions dans la formation des images.

Différents systèmes d'alignement sont connus qui permettent de contrôler par exemple que la pupille de l'oeil et la pupille d'entrée du système d'imagerie sont superposées, à la fois latéralement et axialement.

De façon classique, il est par exemple connu un système d'alignement de l'oeil comprenant des sources d'alignement couplées à une caméra de visualisation de l'oeil. Les sources d'alignement sont imagées en réflexion par le dioptre cornéen de l'oeil pour former des sources secondaires virtuelles. En observant la position des images des sources secondaires au moyen de la caméra de visualisation de l'oeil, on peut procéder au centrage latéral de la pupille de l'oeil par rapport à la pupille d'entrée du système d'imagerie de l'appareil d'imagerie ophtalmologique. Le centrage axial, c'est-à-dire le long d'un axe optique du système d'imagerie, est fait manuellement par un opérateur.

D'autres systèmes d'alignement sont connus qui permettent en plus du contrôle du centrage latéral, un contrôle automatique du centrage axial.

La demande de brevet publiée EP2281500 décrit par exemple un système d'alignement avec deux caméras, permettant par des mesures triangulaires de contrôler à la fois le centrage latéral et le centrage axial de la pupille de l'oeil. Cependant, un tel système d'alignement est plus encombrant et complexe à mettre en oeuvre.

Le brevet délivré US 5,889,576 décrit un appareil ophtalmologique comprenant des moyens de mesure utilisant la réflexion d'un flux lumineux projeté dans l'oeil à travers la pupille et des moyens de contrôle de l'alignement de la position de la pupille de l'oeil latéral et axial. Plus précisément, les moyens de contrôle de l'alignement comprennent des moyens d'imagerie de la partie antérieure de l'oeil, des moyens de projection de cibles d'alignement à distance finie dans l'espace de l'oeil et de cibles d'alignement à distance infinie et des moyens de détection des cibles. Les cibles d'alignement sont réfléchies par la cornée de l'oeil et imagées par les moyens d'imagerie de la partie antérieure l'oeil. L'alignement axial est contrôlé en comparant la distance entre les images des cibles à distance infinie et la distance entre les images des cibles à distance finie Un tel système nécessite notamment l'agencement de collimateurs pour la projection de cibles à distance infinie, ce qui augmente l'encombrement de l'appareil.

La présente description décrit des systèmes et procédés de contrôle de l'alignement qui permettent un contrôle du centrage de la pupille de l'oeil latéral et axial, sans ajout d'éléments optiques supplémentaires. Il est ainsi possible sans augmenter la complexité ou l'encombrement du système d'alignement, de maintenir au cours d'un examen ophtalmologique une superposition entre le centre de la pupille de l'oeil et un point origine d'un référentiel du système d'imagerie de l'appareil d'imagerie ophtalmologique, malgré les mouvements de l'œil.

## Résumé de l'invention

Dans la présente description, le terme « comprendre » signifie la même chose que « inclure » ou « contenir », et est inclusif ou ouvert et n'exclut pas d'autres éléments non décrits ou représentés.

En outre, dans la présente description, le terme « environ » ou « sensiblement » est synonyme de (signifie la même chose que) une marge inférieure et/ou supérieure de 10%, par exemple 5%, de la valeur respective.

Selon un premier aspect, la présente description concerne un procédé de contrôle de l'alignement de l'oeil dans un appareil d'imagerie ophtalmologique, ledit appareil comprenant un système d'imagerie avec un axe optique donné.

Le procédé selon le premier aspect comprend :

- la formation sur un plan de détection bidimensionnel d'au moins une image d'au moins un élément situé dans un plan de la pupille de l'oeil, au moyen d'un élément optique d'imagerie comprenant un axe optique donné, ledit plan de détection étant incliné de telle sorte qu'une normale au plan de détection forme un angle non nul avec ledit axe optique de l'élément optique d'imagerie;
- la détermination d'une position latérale de la pupille de l'oeil par rapport à un point origine d'un référentiel du système d'imagerie dans l'espace de l'oeil, à partir de la position de ladite au moins une image dans ledit plan de détection;

- l'analyse d'un état de focalisation de ladite au moins une image dans au moins deux régions du plan de détection, agencées selon une direction de variation de netteté définie par l'intersection du plan de détection avec un plan formé par une normale au plan de détection et ledit axe optique de l'élément optique d'imagerie;

- la détermination d'une position axiale de la pupille de l'oeil par rapport audit point origine du référentiel du système d'imagerie, à partir de ladite analyse de l'état de focalisation.

Au sens de la présente description, un axe optique du système d'imagerie est défini par une ligne passant sensiblement par le centre d'une pupille dudit système d'imagerie et le centre d'un champ objet du système d'imagerie. Cette ligne peut être une ligne brisée s'il y a dans l'appareil d'imagerie des miroirs de renvoi ou autres éléments de déflexion de la lumière. Une pupille du système d'imagerie peut être définie par un diaphragme physique du système optique, ou bien, dans le cas d'un système d'imagerie par balayage comprenant un ou plusieurs miroirs de balayage, une pupille du système d'imagerie peut être définie par un desdits miroirs de balayage ou par la taille physique d'un faisceau de balayage entre les deux miroirs.

Une pupille d'entrée du système d'imagerie est une pupille conjuguée optiquement de la pupille du système d'imagerie, dans l'espace objet du système optique, c'est-à-dire dans l'espace de l'oeil.

Dans la présente description, on appelle de façon générale « direction axiale » dans un espace donné, une direction colinéaire à l'axe optique du système d'imagerie, considéré dans ledit espace, et « position axiale » une position mesurée selon une direction axiale.

On appellera de façon générale « direction latérale » dans un espace donné, une direction perpendiculaire à l'axe optique du système d'imagerie, considéré dans ledit espace, et « position latérale », une position mesurée selon une direction latérale.

Selon un ou plusieurs exemples de réalisation, le référentiel du système d'imagerie dans l'espace de l'oeil est défini par l'axe optique du système d'imagerie et le plan de la pupille d'entrée du système optique. L'origine du référentiel est le centre de la pupille d'entrée du système d'imagerie.

Selon un ou plusieurs exemples de réalisation, le référentiel du système d'imagerie dans l'espace de l'oeil est défini par l'axe optique du système d'imagerie et un plan conjugué d'un plan d'imagerie du système optique, dans l'espace objet du système d'imagerie. L'origine du référentiel est le centre d'un champ objet du système d'imagerie.

Plus généralement, le référentiel du système d'imagerie dans l'espace de l'oeil peut être défini par l'axe optique du système d'imagerie et un plan prédéterminé dans l'espace objet du système d'imagerie (espace de l'oeil), centré sur l'axe optique.

Selon un ou plusieurs exemples de réalisation, un écart angulaire entre l'axe optique de l'élément optique d'imagerie et l'axe optique du système d'imagerie, dans l'espace de l'oeil, est inférieur à environ 20% des angles maximaux des rayons optiques se propageant dans l'élément optique d'imagerie et/ou un décalage latéral desdits axes optiques est inférieur à 20% des dimensions maximales des faisceaux optiques se propageant dans l'élément optique d'imagerie.

Selon un ou plusieurs exemples de réalisation, l'axe optique de l'élément optique d'imagerie est sensiblement confondu dans l'espace de l'oeil avec l'axe optique du système d'imagerie, c'est-à-dire qu'un écart angulaire entre les deux axes est inférieur à environ 10%, avantageusement inférieur à environ 5% des angles maximaux des rayons optiques se propageant dans le élément optique d'imagerie et/ou qu'un décalage latéral des deux axes optiques est inférieur à 10%, avantageusement inférieur à environ 5% des dimensions maximales des faisceaux optiques se propageant dans l'élément optique d'imagerie.

Selon un ou plusieurs exemples de réalisation, ladite au moins une image d'au moins un élément situé dans un plan de la pupille de l'oeil est formée par ledit élément optique d'imagerie et par au moins une partie des éléments optiques formant le système d'imagerie.

Selon un ou plusieurs exemples de réalisation, la formation d'au moins une image comprend la formation d'images d'au moins deux sources secondaires ponctuelles ou quasi-ponctuelles virtuelles situées dans le plan de la pupille de l'oeil, les images desdites au moins deux sources virtuelles étant décalées, dans ledit plan de détection, dans la direction de variation de netteté.

Selon un ou plusieurs exemples de réalisation, lesdites sources secondaires ponctuelles ou quasi-ponctuelles virtuelles sont formées par les images en réflexion par la cornée d'au moins deux sources lumineuses primaires, lesdites deux sources primaires étant configurées pour éclairer la cornée avec des faisceaux d'illumination dont les projections dans un plan contenant ledit axe optique de l'élément optique d'imagerie et la normale au plan de détection font un angle compris entre environ 20° et environ 180°, avantageusement entre environ 30° et environ 80°.

Par exemples, les sources primaires comprennent des diodes électroluminescentes (ou « LED » selon l'abréviation de l'expression anglosaxonne « Light Emitting Device ») ou des sorties de fibres optiques éclairées en entrée par une ou plusieurs source(s) lumineuse(s).

Selon un ou plusieurs exemples de réalisation, la bande spectrale d'émission des sources primaires est dans le proche infrarouge, comprise par exemple entre environ 700 nm et environ 1100 nm, avantageusement comprise entre environ 800 nm et environ 1000 nm. Selon un ou plusieurs exemples de réalisation, la formation d'au moins une image comprend la formation d'un élément structurel naturel de la pupille de l'oeil, par exemple un élément structurel de l'iris.

L'analyse d'un état de focalisation de ladite au moins une image dans au moins deux régions du plan de détection, également appelée analyse de la netteté dans la présente description, comprend généralement la détermination d'un ou plusieurs paramètres de nature à mesurer l'état de focalisation de ladite au moins une image dans lesdites au moins deux régions.

Par exemple, dans le cas d'une image d'un élément structurel de la pupille de l'oeil, l'analyse de l'état de focalisation peut comprendre, selon un ou plusieurs exemples de réalisation, la détermination d'un paramètre de netteté de l'image dans au moins deux régions différentes de l'image prises selon la direction de variation de netteté. Un paramètre de netteté est par exemple déterminé au moyen d'un traitement mathématique connu, par exemple un filtre de Sobel ou tout autre algorithme utilisé pour l'analyse de la netteté, par exemple des algorithmes connus dans les systèmes d'imagerie autofocus. Pour augmenter la robustesse de la détermination de la position axiale de la pupille de l'oeil, l'analyse de netteté peut être réalisée sur l'ensemble de l'image découpée en régions correspondant à des positions différentes selon la direction de variation de netteté.

Dans le cas d'une image d'un élément structurel de la pupille de l'oeil, la détermination de la position centrale de la pupille de l'œil peut comprendre une mesure de la position du centre de la pupille à l'aide d'une méthode connue de détection de pupille.

Dans le cas de la formation d'images d'au moins deux sources secondaires ponctuelles ou quasi-ponctuelles virtuelles situées dans le plan de la pupille de l'oeil, lesdites images étant décalées dans la direction de variation de netteté, l'analyse de l'état de focalisation peut comprendre, selon un ou plusieurs exemples de réalisation, la détermination d'un ou plusieurs paramètres de caractérisation de la position et d'une dimension desdites images. Ces paramètres comprenant par exemple et de façon non limitative : la position du centre de chacune desdites images, une dimension de chacune desdites images, comme par exemple un diamètre, un rayon ou une valeur moyenne du diamètre ou du rayon, ou un paramètre lié à la dimension de chacune desdites images, par exemple une mesure de l'intensité moyenne ou de l'intensité maximale de chacune desdites images. A noter que les paramètres d'intensité, dépendant de la puissance des sources primaires, sont moins adaptés.

Selon un ou plusieurs exemples de réalisation, le procédé selon le premier aspect comprend en outre une étape de calibration préalable, ladite calibration comprenant la détermination d'une loi donnant lesdites positions axiale et latérale de la pupille de l'oeil dans le référentiel du système d'imagerie en fonction dudit état de focalisation de ladite au moins une image dans lesdites au moins deux régions du plan de détection.

Ainsi, selon un ou plusieurs exemples de réalisation, ladite loi de calibration peut comprendre la détermination d'une loi donnant lesdites positions axiale et latérale de la pupille de l'œil dans le référentiel du système d'imagerie en fonction d'un paramètre de netteté déterminé dans la direction d'analyse de netteté, par exemple un maximum de netteté. Dans le cas de formation d'images de sources secondaires, ladite loi de calibration peut comprendre la détermination d'une loi donnant lesdites positions axiale et latérale de la pupille de l'oeil dans le référentiel du système d'imagerie en fonction de paramètres de caractérisation de positions et/ou de dimensions des images.

La déposante a montré qu'un procédé de contrôle de l'alignement selon le premier aspect, mettant en oeuvre une analyse d'un état de focalisation d'au moins une image sur un plan de détection incliné, permet une détermination robuste et automatique de la position de la pupille de l'oeil dans un référentiel donné du système d'imagerie, à la fois latérale et axiale. Selon un ou plusieurs exemples de réalisation, un angle d'inclinaison du plan de détection défini entre une direction normale au plan de détection et l'axe optique de l'élément optique d'imagerie est compris entre environ 2° et environ 30°, avantageusement entre environ 5° et environ 15°. La déposante a montré qu'un tel angle d'inclinaison du plan de détection permettait un bon compromis pour la détermination des positions latérale et axiale de la pupille de l'oeil.

Selon un ou plusieurs exemples de réalisation, le procédé selon le premier aspect comprend en outre un centrage latéral et/ou un centrage axial de la pupille de l'œil par rapport audit point origine du référentiel du système d'imagerie, à partir desdites positions latérale et axiale de la pupille de l'oeil, préalablement déterminées. Ainsi, on détermine dans un premier temps les positions latérales et axiales de la pupille de l'oeil, puis on procède à un centrage de la pupille de l'oeil.

Par exemple, les centrages axial et/ou latéral de la pupille de l'oeil sont obtenus par déplacement de tout ou partie de l'appareil d'imagerie ophtalmologique ou par déplacement de support solidaire de la tête du patient.

Selon un ou plusieurs exemples de réalisation, le procédé selon le premier aspect comprend en outre un centrage itératif et continu comprenant une succession de corrections partielles des décalages mesurés de la pupille de l'oeil ; un tel centrage itératif et continu permet d'améliorer la stabilité du centrage de la pupille de l'oeil. A chaque étape, il peut comprendre un centrage latéral et/ou axial partiel de la pupille de l'oeil comprenant une correction comprise entre environ 40% et environ 60% des décalages latéral et axial de la pupille de l'oeil préalablement déterminés, par exemple environ 50%. Les décalages latéral et/ou axial sont définis respectivement par les positions latérale et/ou axiale de la pupille de l'oeil mesurées par rapport au point origine du référentiel. Le procédé de contrôle de l'alignement de l'oeil peut alors être réitéré sur la base des nouvelles positions de la pupille de l'oeil partiellement corrigées.

Selon un ou plusieurs exemples de réalisation, dans le cas d'une formation d'images d'au moins deux sources secondaires ponctuelles ou quasi-ponctuelles virtuelles situées dans le plan de la pupille de l'oeil, les images desdites au moins deux sources virtuelles étant décalées dans la direction de variation de netteté, le procédé selon le premier aspect comprend en outre un centrage latéral de la pupille de l'oeil par rapport audit point origine du référentiel du système d'imagerie, à partir de la position latérale de la pupille de l'œil déterminée, puis l'analyse de l'état de focalisation après ledit centrage latéral.

La déposante a montré en effet que dans ce cas, la détermination de la position axiale de la pupille de l'oeil dépend de la position latérale de la pupille de l'oeil. On peut ainsi soit corriger la position latérale de la pupille de l'oeil avant de procéder à l'analyse de l'état de focalisation, soit tenir compte à la fois de l'état de focalisation et de la position latérale de la pupille de l'oeil pour déterminer la position axiale de la pupille de l'oeil.

Selon un deuxième aspect, la présente description concerne un système pour le contrôle de l'alignement de l'oeil dans un appareil d'imagerie ophtalmologique comprenant un système d'imagerie avec un axe optique donné, le système pour le contrôle de l'alignement de l'oeil comprenant :

- un élément optique d'imagerie comprenant un axe optique donné, configuré pour former une image d'au moins un élément (115b, 116b) situé dans un plan de la pupille de l'œil ;
- un dispositif d'acquisition d'images comprenant un plan de détection bidimensionnel, ledit dispositif d'acquisition étant configuré pour l'acquisition de ladite au moins une image dudit au moins un élément situé dans le plan de la pupille de l'oeil, ledit plan de détection étant incliné de telle sorte qu'une normale au plan de détection forme un angle non nul avec ledit axe optique de l'élément optique d'imagerie;
- une unité de contrôle configurée pour :

  - la détermination d'une position latérale de la pupille de l'oeil par rapport à un point origine d'un référentiel du système d'imagerie, à partir de la position de ladite au moins une image dans ledit plan de détection;
  - l'analyse d'un état de focalisation de ladite au moins une image dans au moins deux régions du plan de détection, agencées selon une direction de variation de netteté, ladite direction de variation de netteté étant définie par l'intersection du plan de détection avec un plan formé par une normale au plan de détection et ledit axe optique de l'élément optique d'imagerie;
  - la détermination d'une position axiale de la pupille de l'oeil par rapport audit point origine du référentiel du système d'imagerie, à partir de ladite analyse de l'état de focalisation.

Selon un ou plusieurs exemples de réalisation, ledit axe optique de l'élément optique d'imagerie est sensiblement confondu dans l'espace de l'oeil avec ledit axe optique du système d'imagerie.

Selon un ou plusieurs exemples de réalisation, ledit au moins un élément situé dans un plan de la pupille de l'œil comprend au moins deux sources secondaires ponctuelles ou quasi-ponctuelles virtuelles situées dans le plan de la pupille de l'oeil, les images desdites au moins deux sources virtuelles étant décalées, dans ledit plan de détection, dans la direction de variation de netteté.

Selon un ou plusieurs exemples de réalisation, le système selon le deuxième aspect comprend en outre au moins deux sources lumineuses primaires, lesdites deux sources primaires étant configurées pour éclairer la cornée avec des faisceaux d'illumination dont les projections dans un plan contenant ledit axe optique de l'élément optique d'imagerie et la normale au plan de détection font un angle compris entre environ 20° et environ 180°, lesdites sources secondaires ponctuelles ou quasi-ponctuelles virtuelles résultant de l'image par en réflexion la cornée desdites au moins deux sources primaires.

Selon un troisième aspect, la présente description concerne un appareil d'imagerie ophtalmologique comprenant un système d'imagerie avec un axe optique donné et un système pour le contrôle de l'alignement de l'œil selon le deuxième aspect.

Selon un ou plusieurs exemples de réalisation, ledit appareil d'imagerie ophtalmologique est un appareil d'imagerie d'un segment postérieur de l'oeil (e.g. la rétine), par exemple un appareil OCT pour capturer des images tomographiques de segments postérieurs (e.g. rétine) de l'oeil, un appareil d'imagerie du fond de l'oeil (ou « fundus caméra » selon l'expression anglo-saxonne), un ophtalmoscope à balayage laser (SLO) pour capturer des images du fond de l'oeil par balayage d'un faisceau laser à l'aide d'un système optique confocal, etc.

Selon un ou plusieurs exemples de réalisation, ledit appareil d'imagerie ophtalmologique est un appareil d'imagerie d'un segment antérieur de l'oeil (e.g. cornée), par exemple un appareil OCT, un kératomètre, une lampe à fente, etc.

**Brève description des figures**

D'autres avantages et caractéristiques de l'invention apparaîtront à la lecture de la description, illustrée par les figures suivantes :

[Fig. 1A], représente un schéma simplifié illustrant un appareil d'imagerie ophtalmologique équipé d'un exemple de système pour le contrôle de l'alignement de l'oeil selon la présente description ;
[Fig. 1B], représente un schéma illustrant un exemple de système pour le contrôle de l'alignement de l'oeil selon la présente description ;
[Fig. 2] représente des schémas illustrant un exemple de procédé de centrage axial de la pupille de l'oeil, selon la présente description ;

[Fig. 3] représente des schémas illustrant le couplage du centrage latéral et du central axial de la pupille de l'oeil, dans un exemple de procédé de contrôle de l'alignement de l'œil selon la présente description ;

[Fig. 4] représente un schéma illustrant un exemple de détermination du contrôle de l'alignement de l'oeil , dans un exemple comprenant la formation d'images de deux sources secondaires ponctuelles ou quasi-ponctuelles virtuelles résultant de l'image en réflexion par la cornée de deux sources lumineuses primaires ;

[Fig. 5] illustre la formation d'images de quatre sources secondaires ponctuelles ou quasi-ponctuelles virtuelles résultant de l'image en réflexion par la cornée de quatre sources lumineuses primaires, dans un exemple de contrôle de l'alignement de l'oeil selon la présente description.

## Description détaillée de l'invention

Sur les figures, les éléments ne sont pas représentés à l'échelle pour une meilleure visibilité.

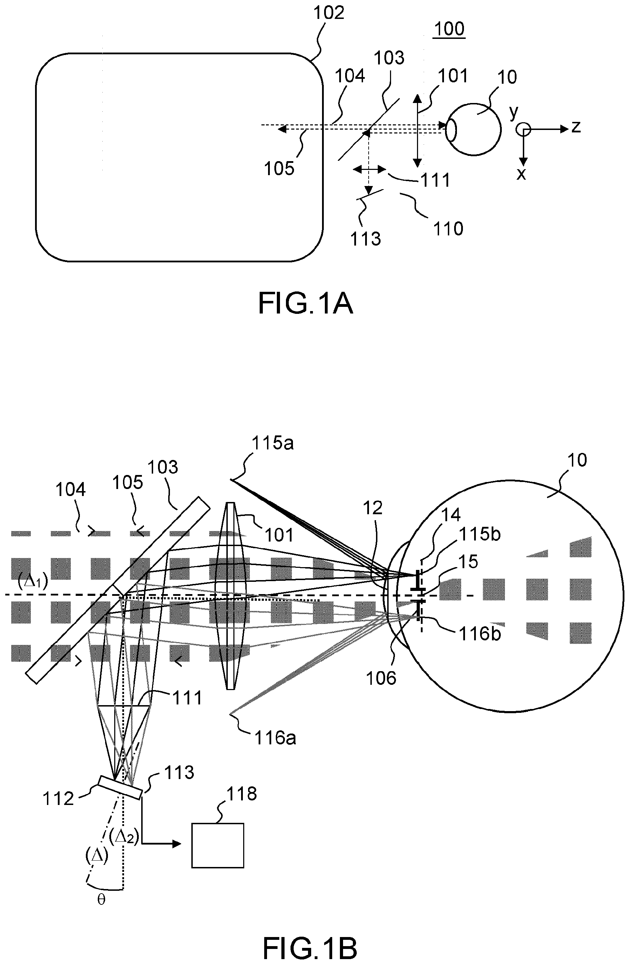

La Fig. 1A représente un schéma simplifié illustrant un exemple d'appareil d'imagerie ophtalmologique 100 équipé d'un système 110 pour le contrôle de l'alignement de l'oeil selon la présente description et la Fig. 1B représente un schéma illustrant un exemple d'un tel système pour le contrôle de l'alignement de l'oeil.

L'appareil d'imagerie ophtalmologique 100 comprend généralement un système d'imagerie 101 avec un axe optique $\Delta_1$ (Fig. 1B) représenté sur les figures le long de l'axe z d'un repère (xyz) défini dans l'espace objet du système d'imagerie, c'est-à-dire l'espace de l'oeil. L'appareil d'imagerie ophtalmologique 100 comprend également une unité d'illumination et de détection 102 dans laquelle se trouve un plan d'imagerie (non représenté).

L'unité d'illumination et de détection 102 est propre à chaque appareil d'imagerie et est adaptée à la partie de l'oeil 10 que l'on cherche à imager, par exemple un segment antérieur de l'oeil, comme la cornée, ou un segment postérieur de l'oeil, comme la rétine. Elle peut comprendre, de façon connue, et en fonction du type d'appareil, des lentilles optiques, des miroirs, des sources de lumières, un ou plusieurs détecteur(s), un ou plusieurs miroir(s) de balayage, un interféromètre (cas d'un appareil OCT), un spectroscope (cas d'un appareil OCT).

Sur les Figs. 1A et 1B seuls sont représentés par souci de simplification, un faisceau d'illumination 104 pour l'éclairage de la partie de l'oeil que l'on cherche à imager et un faisceau 105 rétrodiffusé par ladite partie de l'oeil.

Dans l'exemple illustré sur les Figs. 1A et 1B, le contrôle de l'alignement de la pupille 15 de l'oeil 10 comprend le contrôle de la superposition de ladite pupille 15 avec la pupille d'entrée 106 du système d'imagerie 101 de l'appareil d'imagerie. Bien que représenté de façon schématique sur la Fig. 1B par un simple objectif, le système d'imagerie 101 peut comprendre un ou plusieurs éléments optiques réfractifs ou réfléchissants, l'ensemble de ces éléments optiques se comportant comme un objectif équivalent de focale donnée. Par « superposition des pupilles », on entend que le plan de la pupille 106 du système d'imagerie est sensiblement confondu avec le plan 14 de la pupille de l'oeil et que les centres des pupilles 106, 15 sont sensiblement superposés. Cette configuration s'applique notamment dans le cas d'appareils d'imagerie ophtalmologiques configurés pour l'imagerie de la rétine.

La présente description n'est cependant pas limitée à cette configuration. De manière générale, le contrôle de l'alignement de la pupille 15 de l'oeil 10 pourra comprendre le contrôle de la superposition du centre de la pupille 15 de l'oeil avec un point origine o d'un référentiel (o, x, y, z) du système d'imagerie dans l'espace de l'oeil, le référentiel étant défini par l'axe optique $\Delta_1$ du système d'imagerie et un plan prédéterminé dans l'espace objet du système d'imagerie (espace de l'oeil), centré sur l'axe optique $\Delta_1$.

Ainsi par exemple, dans le cas d'un appareil pour l'imagerie d'un segment antérieur de l'oeil (par exemple la cornée), par exemple de type kératomètre, OCT ou appareil de mesure de l'angle irido-cornéen, le référentiel pourra être défini par l'axe optique $\Delta_1$ du système d'imagerie et un plan objet du système d'imagerie, c'est à dire un plan conjugué optiquement avec un plan d'imagerie, dans l'espace de l'oeil, centré sur l'axe optique $\Delta_1$. Le système 110 pour le contrôle de l'alignement de la pupille 15 de l'oeil 10 comprend dans l'exemple de la Fig. 1B, un élément optique d'imagerie 111 avec un axe optique $\Delta_2$ sensiblement confondu dans l'espace de l'oeil avec l'axe optique $\Delta_1$ du système d'imagerie. Plus généralement, on pourra accepter un écart angulaire entre l'axe optique $\Delta_2$ de l'élément optique d'imagerie et l'axe optique $\Delta_1$ du système d'imagerie, dans l'espace de l'oeil, inférieur à environ 20% des angles maximaux des rayons optiques se propageant dans l'élément optique d'imagerie et/ou un décalage latéral desdits axes optiques inférieur à 20% des dimensions maximales des faisceaux optiques se propageant dans l'élément optique d'imagerie.

Le système 110 comprend en outre un dispositif d'acquisition d'images 112 avec un plan de détection 113 bidimensionnel. Selon la présente description, le plan de détection 113 est incliné autour d'un axe de tilt perpendiculaire à l'axe optique de l'élément optique d'imagerie de telle sorte qu'une normale $\Delta$ au plan de détection forme un angle non nul $\theta$ avec l'axe optique de l'élément optique d'imagerie. Par exemple, un angle d'inclinaison $\theta$ du plan de détection est compris entre environ 2° et environ 30°, avantageusement entre environ 5° et environ 15°.

Par ailleurs, comme cela sera décrit plus en détails par la suite, le dispositif d'acquisition 112 est configuré pour l'acquisition d'au moins une image d'au moins un élément situé dans un plan de la pupille 15 de l'oeil et formée par l'élément optique d'imagerie 111. Dans l'exemple illustré que la Fig. 1B, ladite acquisition d'au moins une image comprend l'acquisition d'images de sources secondaires virtuelles 115b, 116b, qui résultent de la formation d'images en réflexion par la cornée 12 de l'oeil, de sources primaires 115a, 116a. Dans l'exemple illustré sur les Figs. 1A et 1B, l'appareil d'imagerie ophtalmologique 100 comprend en outre un élément séparateur de faisceau 103 configuré pour séparer les voies d'illumination et de détection de l'appareil d'imagerie d'une part et la voie de détection du système 110 pour le contrôle de l'alignement de la pupille d'autre part.

L'élément séparateur 103 comprend par exemple un élément optique semi-réfléchissant, un traitement optique dichroïque, un séparateur de polarisation, ou une surface optique non traitée permettant une réflexion spéculaire de 4% par surface.

Dans l'exemple de la Fig. 1B, l'élément séparateur de faisceau 103 est agencé en aval du système d'imagerie 101. Ainsi, ladite au moins une image d'au moins un élément situé dans un plan de la pupille de l'oeil est formée sur le plan de détection 113 du dispositif d'acquisition 112 par l'élément optique d'imagerie 111 et par le système d'imagerie 101. Dans d'autres exemples de réalisation, l'élément séparateur de faisceau 103 peut être agencé en amont du système d'imagerie 101 ou entre des éléments optiques formant le système d'imagerie 101, auquel cas, ladite au moins une image d'au moins un élément situé dans un plan de la pupille de l'oeil est formée sur le plan de détection 113 du dispositif d'acquisition 112 seulement par l'élément optique d'imagerie 111 ou par l'élément optique d'imagerie 111 et une partie des éléments optiques formant le système d'imagerie 101.

Le système 110 pour le contrôle de l'alignement de l'oeil comprend en outre une unité de contrôle 118 configurée pour la mise en oeuvre d'étapes de procédés de contrôle de l'alignement selon la présente description, à partir de ladite au moins une image formée sur le plan de détection 113 du dispositif d'acquisition 112, dans cet exemple à partir des images des sources secondaires virtuelles 115b, 116b.

De façon générale, l'unité de contrôle 118 à laquelle il est fait référence dans la présente description peut comprend une ou plusieurs entités physiques, par exemple un ou plusieurs ordinateurs. Lorsque dans la présente description, il est fait référence à des étapes de calcul ou traitement pour la mise en oeuvre notamment d'étapes de procédés, il est entendu que chaque étape de calcul ou traitement peut être mis en oeuvre par logiciel, hardware, firmware, microcode ou toute combinaison appropriée de ces technologies. Lorsqu'un logiciel est utilisé, chaque étape de calcul ou traitement peut être mise en oeuvre par des instructions de programme d'ordinateur ou du code logiciel. Ces instructions peuvent être stockées ou transmises vers un support de stockage lisible par l'unité de contrôle et/ou être exécutées par l'unité de contrôle afin de mettre en oeuvre ces étapes de calcul ou traitement.

L'unité de contrôle 118 peut être reliée à un interface utilisateur (non représentée sur la Fig. 1B) et/ou être connectée à des éléments de l'unité d'illumination et de détection 102 de l'appareil d'imagerie.

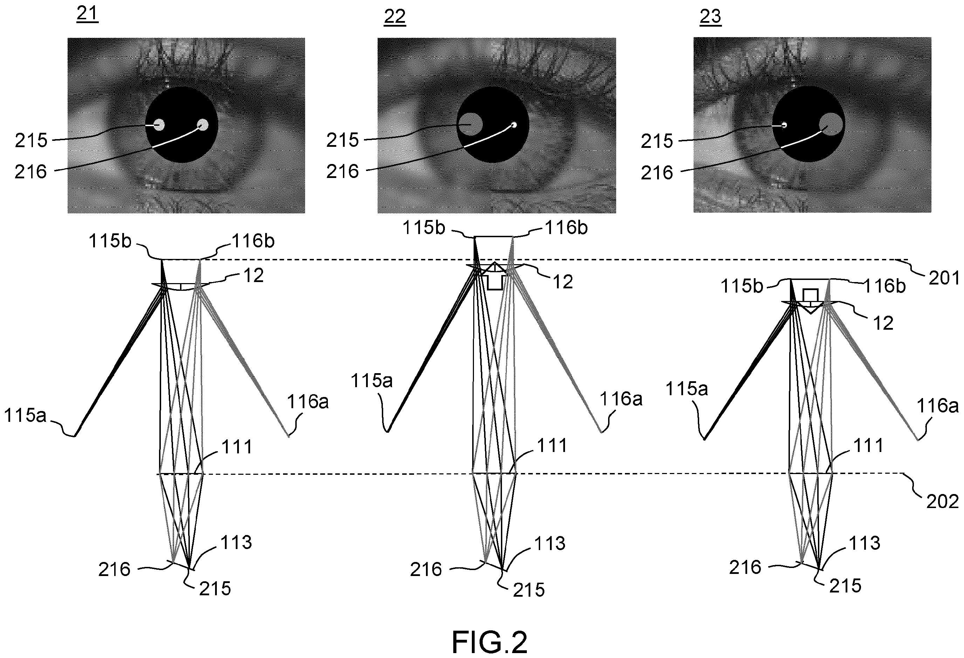

Les Figs 2 à 4 illustrent des exemples d'étapes d'un procédé de contrôle de l'alignement de l'oeil au moyen d'un système de contrôle de l'alignement de l'oeil selon la présente description. Dans ces exemples, par souci de simplicité, seul l'élément optique d'imagerie 111est représenté.

Cependant, comme expliqué précédemment, ladite au moins une image d'au moins un élément situé dans un plan de la pupille de l'oeil peut être formée sur le plan de détection 113 du dispositif d'acquisition également par d'autres éléments optiques, comme le système d'imagerie 101 de l'appareil d'imagerie ophtalmologique (Figs. 1A et 1B), non représenté sur les Figs. 2 à 4.

Dans l'exemple illustré sur les Figs. 2 à 4, le contrôle de l'alignement de l'œil comprend le contrôle de la superposition du centre de la pupille de l'oeil avec un point origine o d'un référentiel (o, x, y, z) du système d'imagerie, défini par l'axe optique $\Delta_1$ du système d'imagerie et un plan prédéterminé dans l'espace objet du système d'imagerie (espace de l'oeil), centré sur l'axe optique $\Delta_1$. Ledit plan prédéterminé est référencé 201 sur les Figs. 2 à 4. Le plan prédéterminé 201 correspond par exemple au plan de la pupille d'entrée du système d'imagerie de l'appareil d'imagerie ophtalmologique.

Comme illustré sur la Fig. 2, le procédé de contrôle de l'alignement de l'œil selon la présente description comprend la formation sur le plan de détection bidimensionnel 113, d'au moins une image d'au moins un élément situé dans le plan de la pupille de l'oeil. Dans cet exemple, la formation d'au moins une image comprend la formation d'images 215, 216 au moyen de l'élément optique d'imagerie 111, de deux sources secondaires virtuelles 115b, 116b, qui résultent de la formation d'images en réflexion par la cornée 12 de l'oeil, de deux sources primaires 115a, 116a.

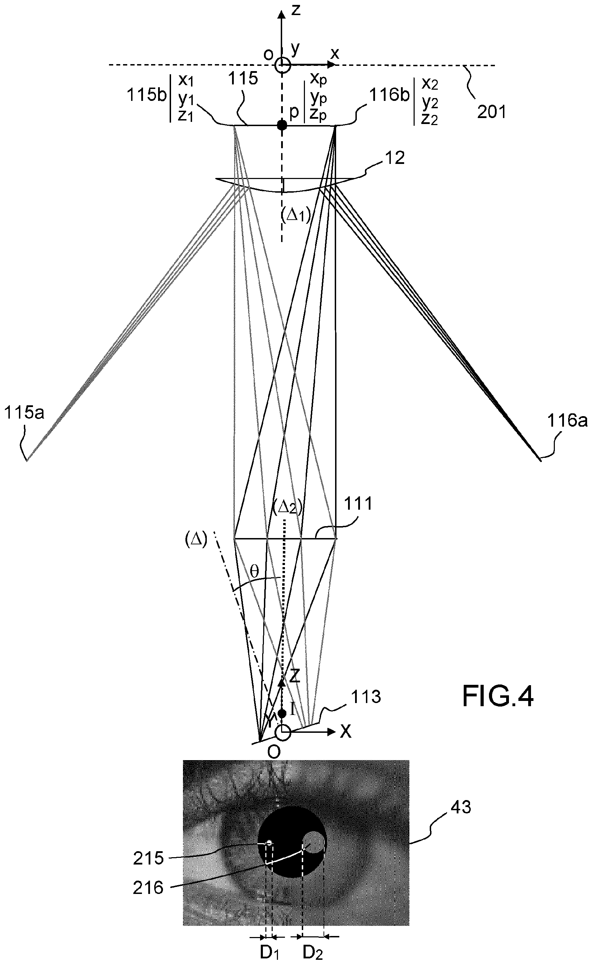

Comme illustré sur La Fig. 4, le plan de détection 113 est incliné de telle sorte qu'une normale Δ au plan de détection 113 forme un angle non nul θ avec ledit axe optique $\Delta_2$ de l'élément optique d'imagerie 111. Du fait de cette inclinaison, un écart du plan pupillaire de l'oeil 114 avec le plan prédéterminé 201 du système d'imagerie, résultera en une différence de netteté accrue entre les images 215, 216 formées par l'élément optique d'imagerie 111.

Ainsi, comme illustré sur la Fig. 2, lorsque les plans pupillaires sont confondus (schéma de gauche), on observe des images 215, 216 sensiblement identiques (image de l'oeil 21). Si le plan pupillaire de l'oeil est décalé axialement par rapport au plan prédéterminé 201 du système d'imagerie (schémas du centre et de droite de la figure), on observe une différence de netteté entre les deux images 215, 216, comme cela est visible sur les images de l'oeil 22, 23.

Il est ainsi possible de déterminer une position axiale de la pupille de l'oeil par rapport au plan 201 de la pupille du système d'imagerie, ou plus généralement par rapport à un point origine o d'un référentiel prédéterminé du système d'imagerie, à partir d'une analyse de netteté des images, ou analyse de l'état de focalisation, selon une direction de variation de netteté. La direction de variation de netteté est définie par l'intersection du plan de détection 113 avec un plan formé par la normale Δ au plan de détection (Fig. 1B) et l'axe optique Δ₂ de l'élément optique d'imagerie.

L'analyse de l'état de focalisation des images 215, 216 comprend, comme cela sera décrit plus en détails par la suite dans un exemple particulier, une mesure de paramètres caractéristiques des positions et des dimensions des images 215, 216, par exemple les positions des centres des images et les diamètres.

En pratique, pour pouvoir déterminer avec précision la position axiale de la pupille de l'oeil à partir de l'analyse de la netteté, on pourra choisir un angle d'inclinaison du plan de détection 113 compris entre environ 2° et environ 30°, avantageusement entre environ 5° et environ 15°.

Par ailleurs, dans l'exemple, tel qu'illustré sur les Figs 2 à 4, où le contrôle de l'alignement de l'oeil comprend la formation d'images au moyen de l'élément optique d'imagerie 111, d'au moins deux sources secondaires virtuelles qui résultent de la formation d'images en réflexion par la cornée 12 de l'oeil, d'au moins deux sources primaires (dans cet exemple 115a, 115b), on pourra agencer les sources primaires pour qu'au moins deux desdites sources primaires ne se trouvent pas alignées selon une direction parallèle à l'axe de tilt du plan de détection. En effet, deux sources primaires ainsi alignées ne permettraient pas de former des images dans le plan de détection, qui soient distinctes dans la direction de variation de netteté.

Avantageusement, lesdites sources primaires pourront être situées de manière à ce que leurs projections respectives dans le plan contenant la normale au plan du détecteur et l'axe optique Δ₂ soient respectivement de part et d'autre de l'axe optique Δ₂, afin de maximiser l'effet de la variation de netteté.

La déposante a montré cependant qu'une différence de netteté entre les images 215, 216 pouvait également résulter d'un défaut de positionnement latéral du centre de la pupille de l'oeil par rapport au centre de la pupille d'entrée du système d'imagerie.

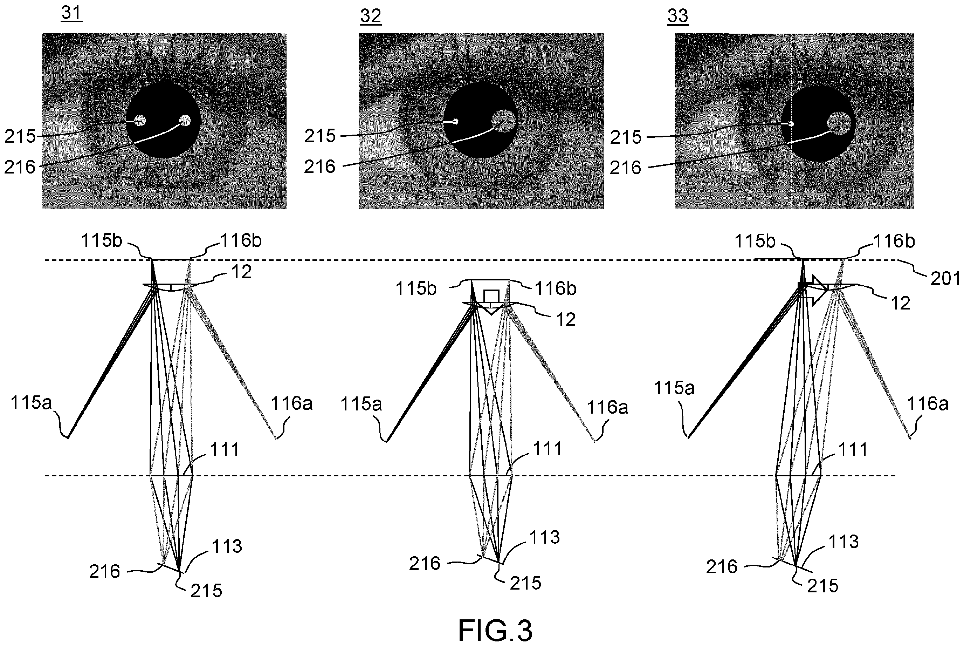

Cet effet est illustré sur la Fig. 3.

Les schémas de gauche et du centre de la Fig. 3 sont similaires aux schémas de gauche et de droite de la Fig. 2. Ainsi, les images de l'oeil 31 et 32 correspondent respectivement à un cas où la pupille de l'oeil est superposée avec le plan prédéterminé 201 (par exemple le plan de la pupille d'entrée du système d'imagerie) et un cas où la pupille de l'oeil est décalée axialement par rapport au plan prédéterminé 201.

Comme cela est visible sur le schéma de droite de la Fig. 3, une image de l'oeil 33 peut être obtenue qui est similaire à l'image 32, mais dans laquelle la pupille de l'oeil se trouve bien dans le plan 201 de la pupille d'entrée du système d'imagerie. La différence de netteté entre les images 215, 216 correspond dans ce cas à un défaut de positionnement latéral de la pupille de l'oeil par rapport au centre de la pupille d'entrée du système d'imagerie.

Ainsi, la détermination de la position axiale de la pupille de l'oeil dans le référentiel du système d'imagerie est faite en tenant compte également de la position latérale de la pupille de l'oeil dans le référentiel.

Ainsi, dans le procédé de contrôle de l'alignement de l'oeil selon la présente description, on pourra, dans un premier temps, déterminer une position latérale de la pupille de l'œil par rapport au centre de la pupille d'imagerie, ou plus généralement par rapport à un point origine o d'un référentiel (o, x, y, z) du système d'imagerie, puis procéder à la détermination d'une position axiale de la pupille de l'oeil par rapport au point origine o du référentiel du système d'imagerie, à partir de l'analyse de la netteté et à partir de la position latérale de la pupille de l'oeil.

Dans des exemples de réalisation, on pourra corriger le centrage latéral de la pupille de l'oeil puis procéder à l'analyse de la netteté en vue de déterminer le positionnement axial de la pupille de l'oeil.

Dans d'autres exemples de réalisation, on pourra déterminer simultanément les positions latérale et axiale de la pupille de l'oeil par rapport au point origine du référentiel du système d'imagerie.

Selon des exemples de réalisation, on pourra alors corriger les décalages mesurés de la pupille de l'oeil par rapport au point origine du référentiel (latéral et/ou axial), par déplacement de tout ou partie de l'appareil d'imagerie, par exemple par déplacement du système d'imagerie, ou par déplacement d'un support solidaire de la tête du patient, par exemple un système d'appui de la tête.

Selon des exemples de réalisation, une correction itérative et continue peut être faire, la correction itérative et continue comprenant une succession de corrections partielles des décalages mesurés et permettant d'améliorer la stabilité du centrage de la pupille de l'oeil. La Fig. 4 représente un schéma illustrant un exemple de détermination des positions axiale et latérale de la pupille de l'oeil par rapport à un référentiel (o, x, y, z), dans un exemple comprenant la formation d'images de deux sources secondaires ponctuelles ou quasi-ponctuelles virtuelles 115b, 116b, résultant de l'image en réflexion par la cornée de deux sources lumineuses primaires. 115a, 116a.

Le référentiel (o, x, y, z) est défini dans l'espace objet du système optique (espace de l'oeil) par l'axe optique Δ₁ du système d'imagerie, colinéaire dans cet exemple à l'axe z du référentiel, et par un plan prédéterminé 201 dans l'espace objet du système d'imagerie (espace de l'oeil), centré sur l'axe optique $\Delta_1$.

On suppose dans cet exemple que les sources primaires 115a et 116a sont agencées dans un plan (plan de la figure) contenant la normale $\Delta$ au plan de détection et l'axe optique $\Delta_2$ de l'élément optique 111, sensiblement confondu avec l'axe optique $\Delta_1$ du système d'imagerie. Par ailleurs, on suppose que les sources primaires 115a et 116a sont agencées de façon symétrique par rapport à l'axe optique $\Delta_2$ de l'élément optique 111. Ces hypothèses ne doivent pas être considérées de façon limitative pour la mise en oeuvre du procédé et visent seulement à illustrer le calcul des positions latérale et axiale de la pupille de l'oeil de façon analytique, dans des hypothèses simplifiées.

Par ailleurs, sur la Fig. 4, seul l'élément optique 111 est représenté pour la formation des images des sources secondaires 115b et 116b. Cependant, en pratique, la formation des images des sources secondaires 115b et 116b peut être obtenue par l'élément optique 111 et tout ou partie du système optique 101 de l'appareil d'imagerie ophtalmologique.

Dans le référentiel (o, x, y, z), les images 115b et 116b des sources primaires 115a et 116a formées en réflexion par la cornée 12 de l'oeil, forment des sources secondaires virtuelles dont les coordonnées sont respectivement $x_1$, $y_1$, $z_1$ et $x_2$, $y_2$, $z_2$. On note $x_p$, $y_p$, $z_p$ les coordonnées du centre p de la pupille de l'oeil.

Comme illustré sur l'image 43, les images 215, 216 des sources secondaires virtuelles 115b et 116b sur le plan de détection incliné 113, par l'élément optique 111, de grandissement y, présentent des diamètres $D_1$, $D_2$.

La position latérale (selon l'axe x) du centre de la pupille de l'oeil, dans le référentiel (o, x, y, z) peut être déterminée par les positions latérales des centres des images 215, 216 formées sur le plan de détection. Plus précisément, dans cet exemple et selon les hypothèses données ci-dessus, $x_p = (x_1 + x_2)\,/2$, $y_p = y_1 = y_2$, $z_p = z_1 = z_2$.

Les positions de l'image de l'oeil dans l'espace image de l'élément optique d'imagerie 111 sont reliées aux positions de l'image de l'oeil dans l'espace objet par le grandissement transverse $\gamma$ de l'élément optique d'imagerie et le grandissement axial $\gamma^2$. On peut donc raisonner de manière équivalente dans l'espace objet ou l'espace image. Plus précisément, dans l'espace image de l'élément optique d'imagerie, les coordonnées des centres des images 215, 216 des sources sont notées $X_1$, $Y_1$, $Z_1$ et $X_2$, $Y_2$, Z, avec $X_1= \gamma\, x_1$, $X_2= \gamma\, x_2$, $Y_1= Y_2 = Y = \gamma\, y$, $Z_1= Z_2 = Z = \gamma^2\, z$.

Sur le plan de détection 113 qui fait un angle $\theta$ avec la normale, on mesure les positions $X'_1$, $X'_2$, $Y'_1$, $Y'_2$ des images 215, 216 des sources (image 43). On a $Y'_1= Y'_2 = Y'$. On en déduit $x_p$ et $y_p$ à partir de $x_1= X_1/\gamma$ $=\cos\theta\; X'_1\,/\,\gamma$, $x_2= \cos\theta\; X'_2\,/\,\gamma$, $x_p= (x_1+x_2)\,/2$, $y_p = Y'\,/\,\gamma$. Reste à déterminer la position axiale $z_p$ de la pupille de l'œil dans le référentiel (o, x, y, z) à partir du grandissement $\gamma$ de l'élément optique 111, de la position latérale du centre de la pupille de l'oeil dans le référentiel (o, x, y, z) et des diamètres respectifs des images 215, 216.

Dans l'espace image, on note I l'image du centre p de la pupille de l'oeil et on note Xi, Yi, Zi les coordonnées de I dans un repère image (O, X, Y, Z). Par souci de simplicité, on suppose que le centre O du repère image et le centre du plan de détection 213 sont confondus.

Dans le cas de deux sources virtuelles 115b, 116b décalées sur l'axe x uniquement d'une quantité identique, leurs images sont, dans l'approximation de l'optique géométrique décalées par rapport à I selon X d'une quantité identique d. Elles ont comme coordonnées respectives Xi - d, Yi, Zi et Xi + d, Yi, Zi.

Dans une approximation de rayons paraxiaux (équivalent à une pupille de sortie de l'élément optique d'imagerie 111 située à l'infini), en considérant un élément optique d'imagerie 111 de nombre d'ouverture N et un angle $\theta$ du capteur petit, les diamètres D1 et D2 des images de sources sur le plan de détection incliné 213 sont :

[MATH 1]

$$D1 = \frac{1}{N}\,|z_i - \theta(x_i - d)|$$

[MATH 2]

$$D2 = \frac{1}{N}\,|z_i - \theta(x_i + d)|$$

On montre avec les équations ci-dessus qu'avec la mesure des positions et des diamètres D1, D2 des images 215, 216 des sources 115b, 116b sur le plan de détection incliné, on peut calculer de manière unique la position de l'image I du centre de la pupille. On peut en déduire la position de la pupille de l'œil dans de référentiel (o, x, y, z) de l'espace objet. Le calcul présenté ci-dessus dans des hypothèses simplifiées pourra être généralisé par l'homme du métier dans d'autres cas plus généraux prenant en compte par exemple un décalage entre les sources secondaires 115b et 116b, une pupille de sortie de l'élément optique d'imagerie 111 à distance finie, un angle $\theta$ plus important, etc.

Par ailleurs, pour une géométrie donnée du système pour le contrôle de l'alignement, on pourra procéder à une calibration théorique ou expérimentale du système en vue de la détermination des positions axiale et latérale de la pupille de l'oeil. La calibration pourra comprendre par exemple la détermination d'une loi qui donne, en fonction des positions et diamètres des images 215, 216 des sources secondaires 115b, 116b, les positions latérale et axiale de la pupille de l'oeil. Cette loi est établie en déterminant au préalable, pour des positions latérale et axiale de la pupille de l'oeil, les positions et diamètres des images 215, 216.

Par ailleurs, le procédé de contrôle d'alignement de la pupille de l'oeil a été décrit dans une configuration dans laquelle deux images 215, 216 sont formées sur le plan de détection, qui sont les conjuguées optiques par optique d'imagerie 111 de deux sources secondaires virtuelles 115b, 116b, qui résultent de la formation d'images en réflexion par la cornée 12 de l'oeil, de deux sources primaires 115a, 116a.

Cependant, le procédé de contrôle d'alignement de la pupille de l'œil selon la présente description peut être mis en oeuvre avec d'autres images formées sur le plan de détection incliné 113.

Par exemple, on pourra utiliser 4 sources primaires au lieu de 2, agencées par exemple selon un carré pour obtenir une redondance et de la robustesse dans la détermination des positions latérale et axiale de la pupille de l'oeil.

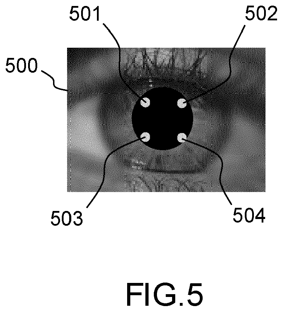

La Fig. 5 illustre ainsi une image de l'oeil 500 sur le plan de détection incliné, avec 4 images 501 - 504 de sources secondaires.

Il est également possible de s'affranchir de l'utilisation de sources primaires en utilisant des éléments structurels naturellement présents dans le plan de la pupille de l'oeil, par exemple de détails de la structure de l'iris de l'oeil dont la netteté va varier selon la direction de netteté, en fonction de la position de la pupille de l'oeil par rapport au point origine du référentiel du système d'imagerie.

Dans ce cas, l'analyse de netteté, ou analyse de l'état de focalisation, peut comprendre la détermination d'un paramètre de netteté de l'image dans au moins deux régions différentes de l'image prises selon la direction de variation de netteté. Un paramètre de netteté est par exemple déterminé au moyen d'un traitement mathématique connu, par exemple un filtre de Sobel ou tout autre algorithme utilisé pour l'analyse de la netteté, par exemple des algorithmes connus dans les systèmes d'imagerie autofocus. Avantageusement, on pourra mesurer l'évolution d'un paramètre de netteté selon la direction de variation de netteté et déterminer la position selon la direction de variation de netteté correspondant au maximum de netteté et en déduite la position axiale de l'oeil.

Bien que décrite à travers un certain nombre d'exemples de réalisation, les procédés et les dispositifs de contrôle de l'alignement de l'oeil selon la présente description comprennent différentes variantes, modifications et perfectionnements qui apparaîtront de façon évidente à l'homme de l'art, étant entendu que ces différentes variantes, modifications et perfectionnements font partie de la portée de l'invention telle que définie par les revendications qui suivent.

**Revendications**

1. Procédé de contrôle de l'alignement de l'oeil (10) dans un appareil d'imagerie ophtalmologique (100) comprenant un système d'imagerie (101) avec un axe optique ($\Delta_1$) donné, le procédé comprenant :

   - la formation sur un plan de détection bidimensionnel (113) d'au moins une image d'au moins un élément (115b, 116b) situé dans un plan (14) de la pupille (15) de l'oeil, au moyen d'un élément optique d'imagerie (111) comprenant un axe optique ($\Delta_2$) donné, ledit plan de détection étant incliné de telle sorte qu'une normale ($\Delta$) au plan de détection forme un angle non nul ($\Theta$) avec ledit axe optique de l'élément optique d'imagerie (111);
   - la détermination d'une position latérale de la pupille de l'oeil par rapport à un point origine (o) d'un référentiel (o, x, y, z) du système d'imagerie dans l'espace de l'oeil, à partir de la position de ladite au moins une image dans ledit plan de détection;
   - l'analyse d'un état de focalisation de ladite au moins une image dans au moins deux régions du plan de détection, agencées selon une direction de variation de netteté définie par l'intersection du plan de détection avec un plan formé par une normale au plan de détection et ledit axe optique de l'élément optique d'imagerie ;
   - la détermination d'une position axiale de la pupille de l'oeil par rapport audit point origine (o) du référentiel du système d'imagerie, à partir de ladite analyse de l'état de focalisation.

2. Procédé de contrôle de l'alignement selon la revendication 1, dans lequel la formation d'au moins une image comprend la formation d'images d'au moins deux sources secondaires ponctuelles ou quasi-ponctuelles virtuelles situées dans le plan de la pupille de l'oeil, les images desdites au moins deux sources virtuelles étant décalées, dans ledit plan de détection, dans la direction de variation de netteté.

3. Procédé selon la revendication 2, dans lequel lesdites sources secondaires ponctuelles ou quasi-ponctuelles virtuelles sont formées par les images en réflexion par la cornée d'au moins deux sources lumineuses primaires, lesdites deux sources primaires étant configurées pour éclairer la cornée avec des faisceaux d'illumination dont les projections dans un plan contenant ledit axe optique de l'élément optique d'imagerie et la normale au plan de détection font un angle compris entre environ 20° et environ 180°.

4. Procédé selon l'une quelconque des revendications 2 ou 3, dans lequel la position axiale de la pupille de l'œil par rapport audit point origine du référentiel du système d'imagerie est déterminé en outre à partir de la position latérale de la pupille de l'oeil.

5. Procédé selon l'une quelconque des revendications précédentes, dans lequel ledit axe optique de l'élément optique d'imagerie (111) est sensiblement confondu dans l'espace de l'oeil avec l'axe optique du système d'imagerie.

6. Procédé selon l'une quelconque des revendications précédentes, comprenant en outre un centrage latéral et/ou axial de la pupille de l'oeil par rapport audit point origine du référentiel (o, x, y, z) du système d'imagerie, à partir de la détermination respectivement de ladite position latérale de la pupille de l'oeil et/ou de la position axiale de la pupille de l'oeil.

7. Procédé selon l'une quelconque des revendications précédentes, comprenant en outre une étape de calibration préalable, ladite calibration comprenant la détermination d'une loi donnant lesdites positions axiale et latérale de la pupille de l'oeil dans le référentiel du système d'imagerie en fonction dudit état de focalisation de ladite au moins une image dans lesdites au moins deux régions du plan de détection.

8. Système (110) pour le contrôle de l'alignement de l'oeil (10) dans un appareil d'imagerie ophtalmologique (100) comprenant un système d'imagerie (101) avec un plan d'imagerie et un axe optique donné, ledit système pour le contrôle de l'alignement de l'œil comprenant :

- un élément optique d'imagerie (111) comprenant un axe optique ($\Delta_1$) donné configuré pour former une image d'au moins un élément (115b, 116b) situé dans un plan de la pupille de l'oeil;
- un dispositif d'acquisition d'images (112) comprenant un plan de détection (113) bidimensionnel, ledit dispositif d'acquisition étant configuré pour l'acquisition de ladite au moins une image dudit au moins un élément (115b, 116b), ledit plan de détection étant incliné de telle sorte qu'une normale ($\Delta$) au plan de détection forme un angle non nul ($\theta$) avec ledit axe optique de l'élément optique d'imagerie;
- une unité de contrôle (118) configurée pour :

- la détermination d'une position latérale de la pupille de l'oeil par rapport à un point origine (o) d'un référentiel (o, x, y, z) du système d'imagerie, à partir de la position de ladite au moins une image dans ledit plan de détection;
- l'analyse d'un état de focalisation de ladite au moins une image dans au moins deux régions du plan de détection, agencées selon une direction de variation de netteté définie par l'intersection du plan de détection avec un plan formé par une normale au plan de détection et ledit axe optique de l'élément optique d'imagerie;
- la détermination d'une position axiale de la pupille de l'oeil par rapport audit point origine du référentiel du système d'imagerie, à partir de ladite analyse de l'état de focalisation.

9. Système selon la revendication 8, dans lequel ledit axe optique de l'élément optique d'imagerie (111) est sensiblement confondu dans l'espace de l'oeil avec ledit axe optique du système d'imagerie.

10. Système selon l'une quelconque des revendications 8 ou 9, dans lequel ledit au moins un élément situé dans un plan de la pupille de l'oeil comprend au moins deux sources secondaires ponctuelles ou quasi-ponctuelles virtuelles situées dans le plan de la pupille de l'oeil, les images desdites au moins deux sources virtuelles étant décalées, dans ledit plan de détection, dans la direction de variation de netteté.

11. Système selon la revendication 10, comprenant en outre au moins deux sources lumineuses primaires, lesdites deux sources primaires étant configurées pour éclairer la cornée avec des faisceaux d'illumination dont les projections dans un plan contenant ledit axe optique de l'élément optique d'imagerie et la normale au plan de détection font un angle compris entre environ 20° et environ 180°, lesdites sources secondaires ponctuelles ou quasi-ponctuelles virtuelles étant formées par les images en réflexion par la cornée desdites au moins deux sources primaires.

12. Appareil d'imagerie ophtalmologique (100) comprenant un système d'imagerie (101) avec un axe optique donné et un système (110) pour le contrôle de l'alignement de la pupille (15) de l'oeil (10), selon l'une quelconque des revendications 8 à 11.

13. Appareil d'imagerie ophtalmologique selon la revendication 12, comprenant en outre des moyens de centrage latéral et/ou axial de la pupille de l'oeil par rapport audit point origine (o) du référentiel (o, x, y, z) du système d'imagerie, à partir de la détermination respectivement de ladite position latérale de la pupille de l'oeil et/ou de la position axiale de la pupille de l'oeil.

**Patentansprüche**

1. Verfahren zum Überprüfen der Ausrichtung des Auges (10) in einer ophthalmologischen Bildgebungsvorrichtung (100), die ein Bildgebungssystem (101) mit einer vorgegebenen optischen Achse ($\Delta_1$) auf-

weist, wobei das Verfahren folgende Schritte aufweist:

- Erzeugen mindestens eines Bildes von mindestens einem Element (115b, 116b), das sich in einer Ebene (14) der Pupille (15) des Auges befindet, auf einer zweidimensionalen Detektionsebene (113) mittels eines abbildenden optischen Elements (111), das eine vorgegebene optische Achse ($\Delta_2$) aufweist, wobei die Detektionsebene derart geneigt ist, dass eine Normale ($\Delta$) der Detektionsebene einen von Null verschiedenen Winkel ($\theta$) mit der optischen Achse des abbildenden optischen Elements (111) einschließt;
- Bestimmen einer seitlichen Position der Augenpupille in Bezug auf einen Ursprungspunkt (o) eines Bezugssystems (o, x, y, z) des Bildgebungssystems im Augenraum, ausgehend von der Position des mindestens einen Bildes in der Detektionsebene;
- Analysieren eines Fokussierungszustands des mindestens einen Bildes in mindestens zwei Bereichen der Detektionsebene, die entlang einer Schärfeänderungsrichtung angeordnet sind, die durch den Schnittpunkt der Detektionsebene mit einer Ebene definiert ist, die durch eine Normale zur Detektionsebene und die optische Achse des abbildenden optischen Elements gebildet wird;
- Bestimmen einer axialen Position der Augenpupille in Bezug auf den genannten Ursprungspunkt (o) des Bezugssystems des Bildgebungssystems aus der genannten Analyse des Fokussierungszustands.

2.  Verfahren zum Überprüfen der Ausrichtung nach Anspruch 1, wobei die Erzeugung von mindestens einem Bild die Erzeugung von Bildern von mindestens zwei virtuellen punktförmigen oder quasi-punktuellen Sekundärquellen aufweist, die in der Ebene der Augenpupille liegen, wobei die Bilder der mindestens zwei virtuellen Quellen in der Detektionsebene in Richtung der Schärfeänderung verschoben sind.

3.  Verfahren nach Anspruch 2, wobei die punktförmigen oder quasi-punktförmigen virtuellen Sekundärquellen durch die von der Hornhaut reflektierten Bilder von mindestens zwei primären Lichtquellen gebildet werden, wobei die beiden primären Quellen derart ausgebildet sind, dass sie die Hornhaut mit Lichtstrahlen anstrahlen, deren Projektionen in eine Ebene, die die optische Achse des abbildenden optischen Elements und die Normale zur Detektionsebene enthält, einen Winkel zwischen etwa 20° und etwa 180° einschließen.

4.  Verfahren nach einem der Ansprüche 2 oder 3, wobei die axiale Position der Augenpupille in Bezug auf den Ursprungspunkt des Bezugssystems des Bildgebungssystems ferner aus der lateralen Position der Augenpupille bestimmt wird.

5.  Verfahren nach einem der vorhergehenden Ansprüche, wobei die optische Achse des abbildenden optischen Elements (111) im Augenraum im Wesentlichen mit der optischen Achse des Bildgebungssystems zusammenfällt.

6.  Verfahren nach einem der vorhergehenden Ansprüche, das ferner eine laterale und/oder axiale Zentrierung der Augenpupille in Bezug auf den genannten Ursprungspunkt des Bezugssystems (o, x, y, z) des Bildgebungssystems aufweist, ausgehend von der Bestimmung der genannten lateralen Position der Augenpupille und/oder der axialen Position der Augenpupille.

7.  Verfahren nach einem der vorhergehenden Ansprüche, das ferner einen vorausgehenden Kalibrierungsschritt aufweist, wobei die Kalibrierung die Bestimmung einer Gesetzmäßigkeit aufweist, die die axiale und die laterale Position der Augenpupille im Bezugssystem des Bildgebungssystems in Abhängigkeit von dem Fokussierungszustand des mindestens einen Bildes in den mindestens zwei Bereichen der Detektionsebene angibt.

8.  System (110) zum Überprüfen der Ausrichtung des Auges (10) in einem ophthalmologischen Bildgebungsverfahren (100), das ein Bildgebungssystem (101) mit einer Abbildungsebene und einer vorgegebenen optischen Achse aufweist, wobei das System zum Überprüfen der Ausrichtung des Auges Folgendes aufweist:

- ein abbildendes optisches Element (111) mit einer vorgegebenen optischen Achse ($\Delta_1$), das derart ausgebildet ist, dass es mindestens ein Element (115b, 116b) abbildet, das in einer Ebene der Pupille des Auges liegt;
- eine Bilderfassungsvorrichtung (112), die eine zweidimensionale Detektionsebene (113) aufweist, wobei die Erfassungsvorrichtung derart ausgebildet ist, dass sie das mindestens eine Bild des mindestens einen Elements (115b, 116b) erfasst, wobei die Detektionsebene derart geneigt ist, dass eine Normale ($\Delta$) der Detektionsebene einen Winkel ungleich Null ($\theta$) mit der optischen Achse des abbildenden optischen Elements bildet;
- eine Steuereinheit (118), die ausgebildet ist, zum:

- Bestimmen einer lateralen Position der Augenpupille in Bezug auf einen Ursprungspunkt (o) eines Bezugssystems (o, x, y, z) des Bildgebungssystems, ausgehend von der Position des mindestens einen Bildes in der Detektionsebene;
- Analysieren eines Fokussierungszustands des mindestens einen Bildes in mindestens zwei Bereichen der Detektionsebene, die gemäß einer Schärfeänderungsrichtung angeordnet sind, die durch den Schnittpunkt der Detektionsebene mit einer Ebene definiert ist, die durch eine Normale zur Detektionsebene und die optische Achse des abbildenden optischen Elements gebildet wird;
- Bestimmen einer axialen Position der Augenpupille in Bezug auf den Ursprungspunkt des Bezugssystems des Bildgebungssystems aus der Analyse des Fokussierungszustands.

9. System nach Anspruch 8, wobei die optische Achse des abbildenden optischen Elements (111) im Augenraum im Wesentlichen mit der optischen Achse des Bildgebungssystems zusammenfällt.

10. System nach einem der Ansprüche 8 oder 9, wobei das mindestens eine Element, das in einer Ebene der Augenpupille liegt, mindestens zwei virtuelle punktförmige oder quasipunktförmige Sekundärquellen aufweist, die in der Ebene der Augenpupille liegen, wobei die Bilder der mindestens zwei virtuellen Quellen in der Detektionsebene in der Richtung der Schärfeänderung verschoben sind.

11. System nach Anspruch 10, ferner aufweisend mindestens zwei primäre Lichtquellen, wobei die beiden primären Lichtquellen derart ausgebildet sind, dass sie die Hornhaut mit Lichtstrahlen anstrahlen, deren Projektionen in eine Ebene, die die optische Achse des abbildenden optischen Elements und die Normale zur Detektionsebene enthält, einen Winkel zwischen etwa 20° und etwa 180° einschließen, wobei die sekundären punktförmigen oder quasi-punktförmigen virtuellen Quellen durch die von der Hornhaut reflektierten Bilder der mindestens zwei primären Lichtquellen gebildet werden.

12. Ophthalmologisches Bildgebungsverfahren (100), aufweisend ein Bildgebungssystem (101) mit einer vorgegebenen optischen Achse und ein System (110) zum Überprüfen der Ausrichtung der Pupille (15) des Auges (10), gemäß einem der Ansprüche 8 bis 11.

13. Ophthalmologisches Bildgebungsverfahren gemäß Anspruch 12, ferner aufweisend Mittel zur lateralen und/oder axialen Zentrierung der Augenpupille in Bezug auf den genannten Ursprungspunkt (o) des Bezugssystems (o, x, y, z) des Bildgebungssystems, ausgehend von der Bestimmung der genannten lateralen Position der Augenpupille und/oder der axialen Position der Augenpupille.

## Claims

1. A method for controlling alignment of the eye (10) in an ophthalmological imaging device (100) comprising an imaging system (101) with a given optical axis ($\Delta_1$), the method comprising:

   - formation, on a two-dimensional detection plane (113), of at least one image of at least one element (115b, 116b) located in a plane (14) of the pupil (15) of the eye, by means of an imaging optical element (111) having a given optical axis ($\Delta_2$), said detection plane being inclined such that a normal ($\Delta$) to the detection plane makes a non-zero angle ($\Theta$) to said optical axis of the imaging optical element (111);
   - determination of a lateral position of the pupil of the eye with respect to a point of origin (o) of a reference frame (o, x, y, z) of the imaging system in the eye space, on the basis of the position of said at least one image in said detection plane;
   - analysis of a state of focus of said at least one image in at least two regions of the detection plane, said regions being arranged in a direction of sharpness variation defined by the intersection of the detection plane with a plane formed by a normal to the detection plane and said optical axis of the imaging optical element;
   - determination of an axial position of the pupil of the eye with respect to said point of origin (o) of the reference frame of the imaging system, on the basis of said analysis of the state of focus.

2. The method for controlling alignment as claimed in claim 1, wherein the formation of at least one image comprises formation of images of at least two point or quasi-point virtual secondary sources located in the plane of the pupil of the eye, the images of said at least two virtual sources being offset, in said detection plane, in the direction of sharpness variation.

3. The method as claimed in claim 2, wherein said point or quasi-point secondary virtual sources are formed by the images in reflection from the cornea of at least two primary light sources, said two primary sources being configured to illuminate the cornea with illuminating beams, the projections of which make an angle comprised between about 20° and about 180° in a plane containing said optical axis of the imaging optical element and the normal to the detection plane.

**4.** The method as claimed in either one of claims 2 and 3, wherein the axial position of the pupil of the eye with respect to said point of origin of the reference frame of the imaging system is further determined on the basis of the lateral position of the pupil of the eye.

**5.** The method as claimed in any one of the preceding claims, wherein said optical axis of the imaging optical element (111) is substantially coincident in the eye space with the optical axis of the imaging system.

**6.** The method as claimed in any one of the preceding claims, further comprising lateral and/or axial centering of the pupil of the eye with respect to said point of origin of the reference frame (o, x, y, z) of the imaging system, on the basis of the determination of said lateral position of the pupil of the eye and/or of the axial position of the pupil of the eye, respectively.

**7.** The method as claimed in any one of the preceding claims, further comprising a prior step of calibration, said calibration comprising determination of a law giving said axial and lateral positions of the pupil of the eye in the reference frame of the imaging system as a function of said state of focus of said at least one image in said at least two regions of the detection plane.

**8.** A system (110) for controlling alignment of the eye (10) in an ophthalmological imaging device (100) comprising an imaging system (101) with an imaging plane and a given optical axis, said system for controlling alignment of the eye comprising:

- an imaging optical element (111) having a given optical axis ($\Delta_1$) and configured to form an image of at least one element (115b, 116b) located in a plane of the pupil of the eye;
- an image-acquiring device (112) comprising a two-dimensional detection plane (113), said acquiring device being configured to acquire said at least one image of said at least one element (115b, 116b), said detection plane being inclined such that a normal ($\Delta$) to the detection plane makes a non-zero angle ($\theta$) to said optical axis of the imaging optical element;
- a control unit (118) configured to:

  - determine a lateral position of the pupil of the eye with respect to a point of origin (o) of a reference frame (o, x, y, z) of the imaging system, on the basis of the position of said at least one image in said detection plane;
  - analyze a state of focus of said at least one image in at least two regions of the detection plane, said regions being arranged in a direction of sharpness variation defined by the intersection of the detection plane with a plane formed by a normal to the detection plane and said optical axis of the imaging optical element;
  - determine an axial position of the pupil of the eye with respect to said point of origin of the reference frame of the imaging system, on the basis of said analysis of the state of focus.

**9.** The system as claimed in claim 8, wherein said optical axis of the imaging optical element (111) is substantially coincident in the eye space with said optical axis of the imaging system.

**10.** The system as claimed in either one of claims 8 and 9, wherein said at least one element located in a plane of the pupil of the eye comprises at least two point or quasi-point virtual secondary sources located in the plane of the pupil of the eye, the images of said at least two virtual sources being offset, in said detection plane, in the direction of sharpness variation.

**11.** The system as claimed in claim 10, further comprising at least two primary light sources, said two primary sources being configured to illuminate the cornea with illuminating beams, the projections of which make an angle comprised between about 20° and about 180° in a plane containing said optical axis of the imaging optical element and the normal to the detection plane, said point or quasi-point virtual secondary sources being formed by the images in reflection from the cornea of said at least two primary sources.

**12.** An ophthalmological imaging device (100) comprising an imaging system (101) with a given optical axis and a system (110) for controlling alignment of the pupil (15) of the eye (10), as claimed in any one of claims 8 to 11.

**13.** The ophthalmological imaging device as claimed in claim 12, further comprising means for centering the pupil of the eye laterally and/or axially with respect to said point of origin (o) of the reference frame (o, x, y, z) of the imaging system, on the basis of the determination of said lateral position of the pupil of the eye and/or of the axial position of the pupil of the eye, respectively.

FIG.1A

FIG.1B

FIG.2

FIG.3

EP 4 099 889 B1

FIG.4

FIG.5

**EP 4 099 889 B1**

## RÉFÉRENCES CITÉES DANS LA DESCRIPTION

**Documents brevets cités dans la description**

- EP 2281500 A

- US 5889576 A